(19) **Europäisches Patentamt / European Patent Office / Office européen des brevets**

(11) **EP 1 362 614 B1**

(12) **EUROPEAN PATENT SPECIFICATION**

(45) Date of publication and mention
of the grant of the patent:
**26.03.2008 Bulletin 2008/13**

(51) Int Cl.:
*A61N 1/372* (2006.01)    *H01Q 9/04* (2006.01)

(21) Application number: **02028759.5**

(22) Date of filing: **23.12.2002**

(54) **Implantable patch antenna**

Implantierbare Streifenleiterantenne

Antenne à plaque implantable

(84) Designated Contracting States:
**CH DE FR IE IT LI**

(30) Priority: **17.05.2002 SE 0201490**

(43) Date of publication of application:
**19.11.2003 Bulletin 2003/47**

(73) Proprietor: **St. Jude Medical AB**
**175 84 Järfälla (SE)**

(72) Inventors:
• **Karlsson, Anders**
**240 10 Dalby (SE)**
• **Abrahamson, Hans**
**113 38 Stockholm (SE)**
• **Lindberg, Magnus**
**172 70 (SE)**

(56) References cited:
**DE-A- 3 150 235**        **US-A- 5 861 019**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

THE BACKGROUND OF THE INVENTION AND PRIOR ART

The present invention relates generally to implantable medical devices that are associated with means for wireless data exchange with extracorporal units. More particularly the invention relates to an antenna arrangement according to the preamble of claim 1 and an implantable device according to the preamble of claim 11.

It is desirable from a patient comfort point of view that an implantable medical device (IMD) can stay in the body for as long time as possible without explantation. Therefore, it is advantageous if the IMD may communicate with the external units while the device is still implanted. Traditionally, status information and measurement parameters have been read out from implanted devices via an inductive telemetry interface. This type of interface is also usable for transportation of data in the opposite direction, for example when adjusting parameters pertaining to the operation of the IMD or when updating the device's program code to a newer version. However, the inductive interface requires a relatively short distance (in the order of centimeters) between the implantanted device and the extracorporal unit with which it communicates. This, in turn, may be inconvenient for the patient as well as impractical for the personnel conducting the procedure. Moreover, the maximum data rate for an inductive interface is relatively low, which results in practical limitations as to the amount of data that can be communicated.

Therefore efforts are now being made in order to find alternative solutions to the inductive telemetry interface for communicating with an IMD. For instance, the American Federal Communications Commission (FCC) and the European Telecommunications Standard Institute (ETSI) have proposed a dedicated radio frequency range in the band 402 - 405 MHz for communication with IMDs. Additionally, the patent literature includes examples of solutions for accomplishing a radio link between an implanted device and an external unit.

U.S. patent No. 6,115,636 describes a telemetry system for implantable devices where the human body functions as a radio antenna for the implanted device. The housing of the device is here used to accomplish a coupling between the patient's body and the device and thus make possible the transmission of a modulated electromagnetic signal.

U.S. patent No. 5,626,630 discloses a telemetry solution involving a quasi-passive implanted transponder and a repeater station to be worn externally by the patient. The repeater station thereby operates as a relay between an IMD and a remote monitoring station to provide a communications link with a high data rate. The transponder includes a couple of microstrip radio antennae, which are resonant at one half of the signal wavelength used.

U.S. patent No. 5,861,019 shows another example of an IMD equipped with a microstrip radio frequency telemetry antenna. Here, the antenna is formed on or within the exterior surface of the device housing. Preferably, the antenna extends over the entire available surface area of the housing and is covered by a radome layer to ensure that the patch is electrically insulated from body fluids and tissue.

Although the above-mentioned solutions generally represent improvements in comparison to an inductive link regarding data rate and communication range, they fail to present a telemetry solution with satisfying power efficiency and sufficiently small physical dimensions of the antenna. The size of the antenna is a major concern because the antenna is to be implanted along with the IMD into a human body. For the best patient comfort, a smallest possible physical size of the antenna is desired. However, low power consumption is at least as important. A change of batteries namely inevitably demands explantation of the device, and should therefore be avoided as long as possible.

The article C. Furse, "Design of an Antenna for Pacemaker Communication," Microwaves and RF, March 2000, pp 73 - 76 describes a microstrip antenna for radio signals at a frequency of 433 MHz. It is proposed that an L-, U- or spiral-shaped patch antenna be arranged on top of a 6 mm thick Teflon substrate. The antenna's physical dimensions are thus limited, however its geometry becomes intricate. Moreover, the design is inclined to cause large ohmic losses and consequently not be particularly power efficient.

SUMMARY OF THE INVENTION

It is therefore an object of the present invention to address these problems and provide an improved implantable antenna solution for an IMD.

According to one aspect of the invention this object is achieved by an antenna arrangement as described initially, which is characterized in that the substrate has a comparatively high relative permittivity and that the substrate extends a well-defined distance outside the volume between the patch plane and the ground plane with respect to at least one second segment of the perimeter. An important advantage attained by such extension of the substrate is that a non-conducting region is thereby obtained outside the patch plane. This, in turn, guarantees that there will only be relatively small electromagnetic losses in this region. Namely, the electric fields closest to the patch are quasi-static and hence very large. In a conducting medium (such as the human body), however, the dissipated power density is proportional to the square of the electric field. This means that if the region closest to the patch plane were occupied with a conducting material a large amount of the power leaving the patch would be used for heating up the region closest to the patch. Naturally, from a power efficiency point of view, this is an undesired effect which is avoided by the proposed antenna arrangement. A high relative permittivity is desirable because the required length of the patch plane is inversely proportional to the square root of the relative permittivity. Consequently, a high relative permittivity makes it possible to accomplish an antenna with small physical dimensions. For example, a substrate of a ceramic material is advantageous, since these materials are available with relative permittivities up to around 100. Alternatively, the substrate may contain a laminated thick-film structure including layers of aluminum oxide. Such substrate can be designed with a relative permittivity of approximately 1000.

According to a preferred embodiment of this aspect of the invention, the substrate overlaps a section of the patch plane along at least one sub-segment of the at least one second segment. An advantage thus attained is that the losses in the dissipative medium represented by the body tissue are further reduced.

According to yet another preferred embodiment of this aspect of the invention, the grounding member includes a conducting plane, which forms a junction with the patch plane along the first segment. A conducting plane connected along the entire length of the first segment results in a uniform distribution of the electric and the magnetic fields over the antenna section. If however, the conducting plane is less extended than the patch plane the resonance frequency for the antenna decreases to a corresponding extent, which may be desirable in some applications.

According to another preferred embodiment of this aspect of the invention, the grounding member is substantially perpendicular to the ground plane. It is not necessary for the antenna function that the grounding member is entirely perpendicular to the ground plane, however such orientation results in a more uniform distribution of the electric and the magnetic fields over the antenna section. Feeding the antenna respective extracting a received signal from the antenna is thereby also facilitated. For instance, the antenna arrangement may include a signal member adapted to transport a radio frequency signal between the patch plane and a radio transceiver. The signal member extends through the ground plane via an electrically insulated feedthrough at a particular distance from the grounding member. The signal member includes an element that is substantially perpendicular to the ground plane and is either electrically insulated from the patch plane or connected to a side of the patch plane, which faces towards the ground plane.

According to another aspect of the invention, this object is achieved by an implantable medical device as described initially, which is characterized in that it includes the proposed antenna arrangement.

According to a preferred embodiment of this aspect of the invention, the antenna arrangement is located in a recess of an outer side of a casing to the device. This is advantageous because a slim device design is thus made possible.

According to a first alternative preferred embodiment of this aspect of the invention, the antenna arrangement is molded into a non-conductive encapsulation, such that the ground plane is electrically insulated from the device's casing. This may be desirable in applications where an absolute control of the ground plane properties is demanded.

According to a second alternative preferred embodiment of this aspect of the invention, the device's casing instead constitutes the ground plane. Again, this vouches for a slim and uncomplicated design of the device.

According to yet another preferred embodiment of this aspect of the invention, the patch plane is tilted with respect to the ground plane, such that the arrangement attains horn antenna properties. This, in turn, results in an antenna with an increased bandwidth, since it facilitates the propagation of the outgoing electromagnetic waves. Moreover, the efficiency of the antenna is enhanced due to an increased radiation resistance. Preferably, the patch plane is tilted such that the distance between the patch plane and the ground plane is shortest at a point where the grounding member connects to the patch plane.

According to still another preferred embodiment of this aspect of the invention, it is presumed that the casing constitutes the ground plane and that the ground plane includes a chamfer. This chamfer is oriented such that the distance between the patch plane and the ground plane extends gradually along a slope of the chamfer and the distance between the ground plane and the patch plane is largest at an edge side being furthest away from the grounding member. Again, this results in an antenna arrangement having properties similar to that of a horn antenna.

A general advantage with the present invention is that simple antenna geometry is provided, which can be manufactured by means of a fairly uncomplicated production process. Furthermore, the proposed antenna geometry results in relatively low ohmic losses.

Although the proposed solution is primarily intended for cardiac devices, such as pacemakers and defibrillators, the invention is equally well applicable to any alternative type of implantable medical devices, for example drug pumps, neurostimulators, gastric stimulators, muscle stimulators and hemodynamic monitors.

BRIEF DESCRIPTION OF THE DRAWINGS

The present invention is now to be explained more closely by means of preferred embodiments, which are disclosed as examples, and with reference to the attached drawings.

Figure 1          schematically shows an IMD which communicates with an extracorporal unit over a wireless interface according to the invention,

Figure 2          illustrates the general design of the proposed antenna arrangement,

Figures 3a-h      show top views of alternative patch plane shapes according preferred embodiments of the invention,

Figure 4          shows a side view of an antenna arrangement according to a first preferred embodiment of the invention,

Figure 5          shows a side view of an antenna arrangement according to a second preferred embodiment of the invention,

Figure 6          shows a side view of an IMD including an antenna arrangement according to a first preferred embodiment of the invention,

Figure 7          shows a side view of an IMD including an antenna arrangement according to a second preferred embodiment of the invention,

Figure 8          shows a side view of an IMD including an antenna arrangement according to a third preferred embodiment of the invention,

Figure 9          illustrates an IMD including an antenna arrangement with a tilted patch plane according to a first preferred embodiment of the invention,

Figure 10         illustrates an IMD including an antenna arrangement with a chamfered ground plane according to a second preferred embodiment of the invention, and

Figure 11         illustrates an IMD including an antenna arrangement with a convex patch plane and a curved ground plane according to a third preferred embodiment of the invention.

DESCRIPTION OF PREFERRED EMBODIMENTS OF THE INVENTION

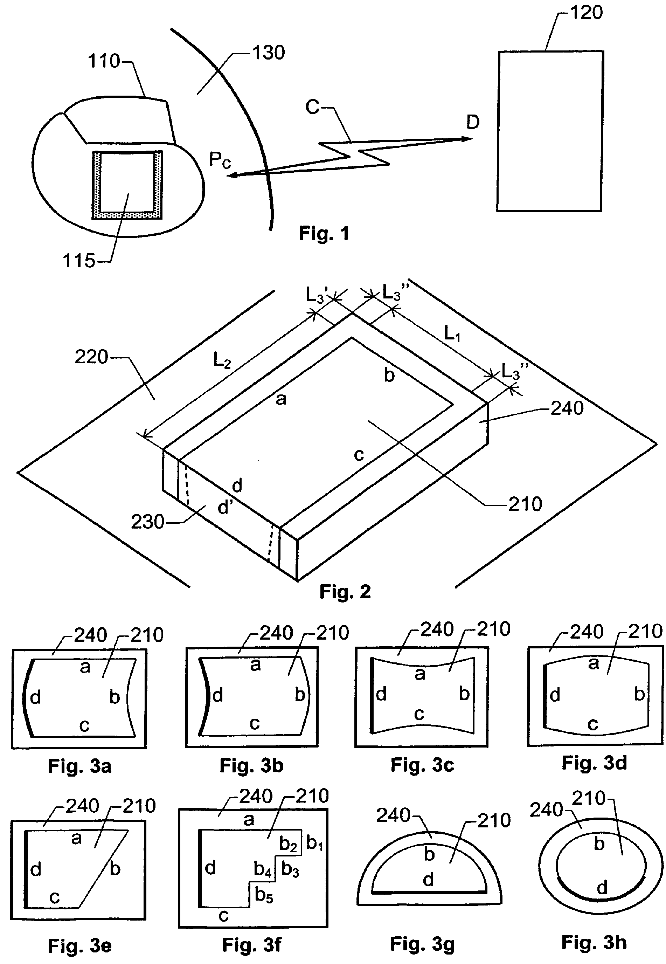

A wireless communication scenario involving an IMD 110 and an extracorporal unit 120 is shown schematically in figure 1. Data D and $P_c$ may here be transported in both directions between the IMD 110 and the extracorporal unit 120 over a radio link C. Typically, parameter settings or updating software $P_c$ is sent from the unit 120 to the IMD 110 while measurement data D is sent in the opposite direction for various monitoring and diagnostic purposes.

According to the invention, an antenna arrangement 115 in the IMD 110 is used to exchange modulated electromagnetic energy with the surrounding transmission medium, i.e. the relevant body tissue 130. Specifically, this means that outgoing radio signals D are coupled through the body 130 and out into the contiguous environment, such as the air. The radio signals D then continue to propagate via the air to the extracorporal unit 120 where they are received, for example by a conventional radio antenna. Correspondingly, incoming radio signals $P_c$ that are received by the IMD 110 have initially been emitted by the unit 120, propagated through the air and penetrated through the body tissue 130 to reach the antenna arrangement 115.

However, transmitting the same radio signals through a lossy medium, such as the human body, and through a much less lossy medium, such as the air, is a far from trivial task. The transmission media's different permittivities must, of course, be considered. The body tissue's properties may be approximated as being close to those of water in respect to permittivity. This means that the wavelength inside the body is roughly 1/9th to 1/8th of the wavelength in free air. For example, if the radio frequency is 403 MHz, the wavelength becomes 0,74 m in free air and around 9,2 cm inside the body.

A patch type of antenna is proposed to be included in the antenna arrangement 115 according to the invention. Such antennae are advantageous because they are easy to build using printed circuit technology. Furthermore, the design results in a comparatively low weight antenna, which has small overall dimensions and is relatively inexpensive to manufacture. Preferably, the antenna design is a planar inverted F-antenna (PIFA), which is resonant at one quarter of a wavelength. I.e. the above example would require a PIFA that is 2,3 cm long. In order to obtain a power efficient antenna, the substrate should have a permittivity that results in a wavelength in the substrate which is approximately equal to the wavelength in the surrounding tissue, i.e. the human body.

The general design of the proposed antenna arrangement 115 is illustrated in figure 2. A patch plane 210 is here adapted to exchange modulated electromagnetic energy with a surrounding transmission medium, i.e. normally body tissue. The depicted patch plane 210 has a general rectangular shape with dimensions $L_1$ and $L_2$ and is thus circumscribed by a perimeter including four separate edges a, b, c and d respectively. However theoretically, any alternative shape of the patch plane 210 is conceivable provided that its physical dimensions are adapted to the specific application. It is namely the patch plane's 210 geometry that determines the antenna's impedance and bandwidth. Various examples of alternative patch plane shapes will be discussed below with reference to figures 3a-h.

Returning to figure 2, a ground plane 220 of an electrically conducting material is located below the patch plane 210. The ground plane 220 has a larger area than the patch plane 210 and is positioned in relation to the patch plane 210, such that a perpendicular projection of the patch plane 210 onto the ground plane 220 falls entirely within the ground plane 220. A dielectric substrate 240 with a comparatively high relative permittivity, say $\varepsilon_r$ = 70 or more, fills the entire volume between the patch plane 210 and the ground plane 220. The substrate 240 also extends a well-defined distance $L_3'$ and/or $L_3''$ outside the volume between the patch plane 210 and the ground plane 220 with respect to at least one the patch plane's 210 edges a, b and c, preferably at least the edge d.

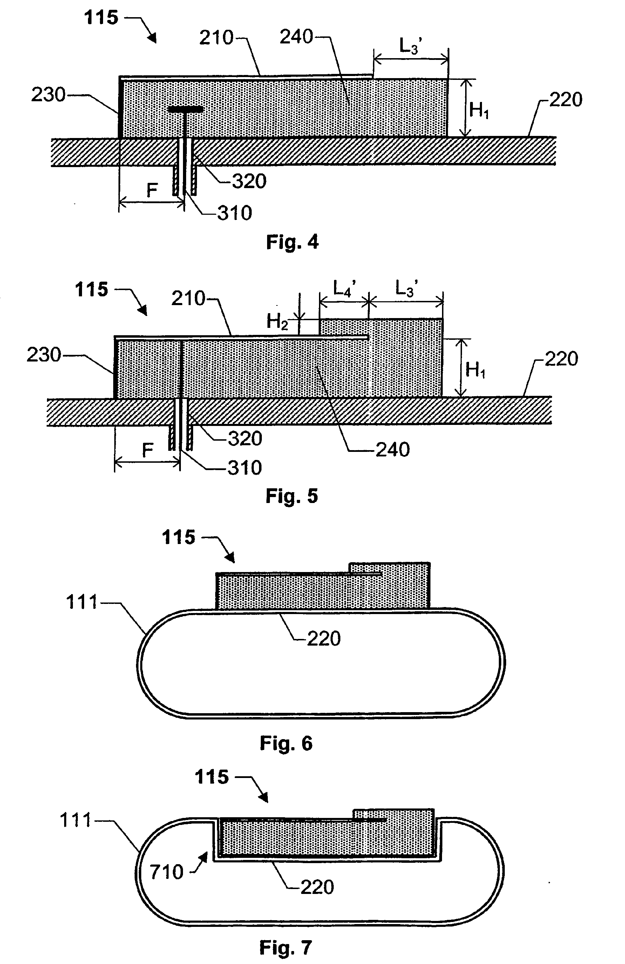

It is important that the substrate 240 extends $L_3'$, $L_3''$ outside the patch plane 210 because thereby a non-conducting region is obtained outside the patch plane 210, where otherwise electrically conducting body tissue would be located. The extension of the substrate 240 guarantees that there will only be a relatively small amount of electromagnetic losses in these regions. The electric fields closest to the patch plane 210 are quasi-static and hence comparatively large. In a conducting medium the dissipated power density is proportional to the square of the electric field. This means that had the region closest to the patch plane 210 been occupied with a conducting material, such as body tissue, a large amount of the power leaving the antenna would be used for heating up the region closest to the patch plane 210. However, the distance $L_3'$, $L_3''$ between the patch plane 210 and the closest conducting region established by the extended substrate 240 accomplishes a volume over which the electric field may decrease without losses. The proposed design is thus very advantageous with respect to power efficiency. Further details pertaining to the relevant dimensions will be discussed below with reference to figures 4 and 5.

According to the above-described embodiment of the invention, a grounding member 230 connects the patch plane 210 electrically to the ground plane 220. Preferably, the grounding member 230 includes a conducting plane, which forms a junction d' with the patch plane 210 along a substantial part of one of the patch plane's edge d. It is generally most preferred if the conducting plane of the grounding member 230 extends along the entire length of the edge d, i.e. a distinct segment of the patch plane's 210 perimeter. A conducting plane whose width equals the patch plane's 210 edge d namely results in a uniform distribution of the electric and the magnetic fields over the antenna section. However, the conducting plane of the grounding member 230 need only extend along a sub-segment of the edge d. If however, the conducting plane is less extended than the patch plane the resonance frequency for the antenna decreases to a corresponding extent, which may be desirable in some applications. Figure 2 shows a grounding member 230 with a shortened conducting plane by means of dashed lines. Note that the conducting plane edges may have arbitrary orientation in relation to the ground plane 220.

As already mentioned, the dielectric substrate 240 preferably has a high relative permittivity. The required length $L_2$ of the patch plane 210 is namely inversely proportional to the square root of the substrate's 240 relative permittivity. Consequently, a high relative permittivity reduces the patch plane's 210 physical dimensions $L_1$ and $L_2$. A ceramic material may be used to accomplish a substrate 240 with a moderate relative permittivity, in the order of 100. If higher values of the relative permittivity are required, the substrate 240 should preferably include a laminated thick-film structure, for instance with layers of aluminum oxide. Thereby, relative permittivities up to 1000 may be accomplished.

Figures 3a-h illustrate examples of alternative patch plane 210 shapes according embodiments of the invention. The first four figures 3a-d represent patch plane 210 shapes that result in comparatively narrowband antennae. Namely, the perpendicular distance between the perimeter segment d being attached to the grounding member and an opposite non-grounded perimeter segment is here constant. Figures 3e-h, however, represent patch plane 210 shapes that result in antennae, which are operable over a broader bandwidth because here the perpendicular distance between the perimeter segment d being attached to the grounding member and an opposite non-grounded perimeter segment varies. For example, the patch plane 210 shape shown in figure 3f provides an antenna adapted to operate over three distinct frequency bands, which are given by the perpendicular distances between the perimeter segment d and the perimeter segments $b_1$, $b_3$ and $b_5$ respectively, whereas the patch plane 210 shape shown in figure 3e provides an antenna adapted to operate over one comparatively wide frequency band.

According to a preferred embodiment of the invention, the grounding member 230 is substantially perpendicular to the ground plane 220. This namely results in a uniform distribution of the electric and the magnetic fields over the antenna section. Moreover, feeding the antenna and extracting a received signal from the antenna is facilitated by such design. This matter will be discussed in further detail below with reference to figures 4 and 5.

Figure 4 shows a side view of an antenna arrangement 115 according to a first preferred embodiment of the invention. The arrangement 115 includes a signal member 310, which is adapted to transport a radio frequency signal between the patch plane 210 and a radio transceiver. I.e. the signal member 310 may be used either to feed a radio signal to the patch plane 210 or to extract a received radio signal there from. The signal member 310 has a cylindrical element, which is substantially perpendicular to the ground plane 220 and extends through the ground plane 220 via an electrically insulated feedthrough 320. The feedthrough 320 is positioned at a distance F from the grounding member 230, where F is selected with respect to the substrate 240 and the properties of the radio signal used, such that a desired impedance of the antenna is obtained. In this embodiment, the signal member 310 ends with a radiating element, which is electrically insulated from the patch plane 210.

The substrate 240 has a thickness $H_1$ and extends a distance $L_3'$ outside the edge b of the patch plane 210. According to a preferred embodiment of the invention, the distance $L_3'$ is proportional to the length $L_2$ of the patch plane 210, such that $L_3'$ lies in the interval 10-30% of $L_2$, and preferably around 20% of $L_2$. According to a preferred alternative of this embodiment of the invention, the substrate 240 also extends a distance $L_3''$ outside the edges a and c of the patch plane 210 (see figure 2). The distance $L_3''$ is likewise proportional to the length $L_2$, such that $L_3''$ lies in the interval 10-20% of $L_2$, preferably at least 15%.

The specific relationships $L_3'$-to-$L_2$ and $L_3''$-to-$L_2$ can be explained as follows. The efficiency of a transmitting an antenna operating in air is defined by the ratio between the power delivered to the antenna and the power radiated from the antenna. The sum of the losses in the substrate and in the conducting parts determines the antenna's efficiency. For an implanted transmitting antenna, however, it is relevant to define the overall efficiency of the antenna system as the ratio between the power delivered to the antenna and the power radiated from the body. Thus, the efficiency is now determined both by the losses in the antenna and in the lossy medium represented by the body tissue. The ohmic power loss density $P_{loss}$ (W/m$^3$) in a conductive medium is given by the expression:

$$P_{loss} = \frac{1}{2} J \cdot E^* = \frac{1}{2} \sigma |E|$$

where $\sigma$ is the conductivity of the medium and E is the electric field. A tissue consisting of muscles has a relatively high conductivity ($\sigma \approx 1{,}4$ at a frequency around 400 MHz including dielectric losses). Hence, the efficiency of the antenna system becomes rather low if the antenna is surrounded by muscle tissue. As is apparent from the above expression, the power loss density $P_{loss}$ is high in regions where the electric field is high. The electric field shows high values in the near zone of the patch plane, since in that region the field is dominated by the non-radiating near field. In the PIFA-case, the near zone is the region close to the outside of the non-grounded segments of the patch plane perimeter (i.e. for example the edges a, b and c in the figure 2).

Figure 5 shows a side view of an antenna arrangement 115 according to a second preferred embodiment of the invention. In this embodiment, the signal member 310, is electrically connected to the side of the patch plane 210, which faces towards the ground plane 220. The signal member 310 is also here substantially perpendicular to the ground plane 220 and extends through the ground plane 220 via an electrically insulated feedthrough 320. The feedthrough 320 is positioned at a distance F from the grounding member 230, where F is selected with respect to the substrate 240 and the properties of the radio signal used, such that a desired impedance of the antenna is obtained.

The substrate 240 has a thickness $H_1$ and does here not only extend a distance $L_3'$ outside the edge b of the patch plane 210, however it also overlaps $L_4'$ a section of the patch plane 210 along at least one edge, for example the edge b. In analogy with the embodiment of the invention described above with reference to the figures 2 and 3, the distance $L_3'$ is preferably proportional to the length $L_2$ of the patch plane 210. The same is basically true for the overlap $L_4'$, however it is sufficient if $L_4'$ corresponds to 10% of $L_2$. The thickness $H_2$ of the overlap should correspond to at least 5% of the length $L_2$ of the patch plane 210. In any case, the overlap assists in further reducing the losses in the dissipative medium represented by the body tissue.

The features of the signal member 310 being electrically connected to the patch plane 210 (as shown in figure 5) respective being insulated from the patch plane 210 (as shown in figure 4) and the substrate 240 overlapping $L_4'$, $H_2$ a section of the patch plane 210 (as shown in the figure 4) respective merely extending a well-defined distance $L_3'$ (as shown in the figure 4) may be combined arbitrarily. The specific combinations of these features as shown in figures 4 and 5 have merely been chosen for illustrating purposes.

Figure 6 shows a side view of an IMD including an antenna arrangement 115 according to a first preferred embodiment of the invention. The arrangement 115 is located on the outer surface of an IMD casing, such that the casing 111 constitutes the ground plane 220. A straightforward design being relatively inexpensive to manufacture is thus made possible.

Figure 7 shows a side view of an IMD including an antenna arrangement 115 according to a second preferred embodiment of the invention. Also here the casing 111 to the device constitutes the ground plane 220. However, the arrangement 115 is now located in a recess 710 of an outer side of the casing 111. This type of design is often preferable, since due to the fact the electric field decreases with increasing substrate thickness, a relatively thick antenna is also comparatively power efficient. By mounting antenna arrangement 115 in the recess 710 it is thus possible to have a rather thick antenna with a high efficiency and at the same time accomplish a slim device.

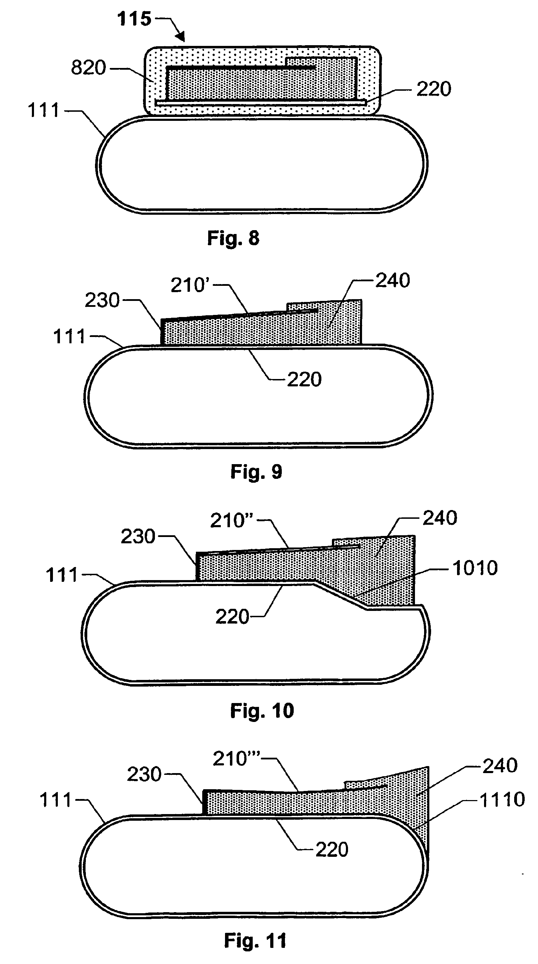

Figure 8 shows a side view of an IMD including an antenna arrangement 115 according to a third preferred embodiment of the invention, where the entire arrangement 115 is molded into a non-conductive encapsulation 820. The encapsulation 820 insulates a ground plane 220 in the arrangement 115 electrically from the casing 111 to the device. Thereby a complete control of the ground plane's 810 properties can be obtained.

Figure 9 illustrates an IMD including an antenna arrangement 115 with a tilted patch plane 210' according to a first preferred embodiment of the invention. Again, the casing 111 to the device constitutes the ground plane 220. The patch plane 210', however, is tilted with respect to the ground plane 220, such that the patch plane 210' is parallel to the ground plane 220 exclusively in one dimension. Hence, the arrangement 115 attains signal properties that resemble those of a horn antenna. For instance, the bandwidth increases. The radiation resistance also raises, which results in a decreased electric field and an enhanced power efficiency.

Preferably, the patch plane 210' is tilted such that the distance between the patch plane 210' and the ground plane 220 is shortest at a point where a grounding member 230 connects to the patch plane 210'.

Figure 10 illustrates an IMD including an antenna arrangement 115 with a chamfered ground plane 220 according to a second preferred embodiment of the invention. Again, the casing 111 to the device constitutes the ground plane 220. Although not actually necessary in this embodiment the patch plane 210" is also tilted with respect to the ground plane 220, such that the patch plane 210" is parallel to the ground plane 220 exclusively in one dimension. More important however, a portion of the casing 111, which constitutes the ground plane 220 here includes a chamfer 1010. This chamfer 1010 is oriented such that the distance between the patch plane 210" and the ground plane 220 extends gradually along a slope of the chamfer 1010. Furthermore, the chamfer 1010 is placed such that the distance between the ground plane 220 and the patch plane 210" is largest at the patch plane edge (edge b in figure 2) being furthest away from a grounding member 230. This design accentuates the horn antenna properties and the thus attained advantages mentioned above.

Figure 11 illustrates an IMD including an antenna arrangement 115 with a convex patch plane 210''' and a curved ground plane 220 according to a third preferred embodiment of the invention. Here, the patch plane 210''' has a general curved profile with a convex face towards the ground plane 220. Again, the casing 111 to the device constitutes the ground plane 220. In this embodiment, the substrate 240 extends over the curved side 1110 of the casing 111, such that the substrate 240 forms a tapered section towards the curved side 1110, which is furthest away from a grounding member 230. Thereby, an alternative horn antenna design is accomplished.

The term "comprises/comprising" when used in this specification is taken to specify the presence of stated features, integers, steps or components. However, the term does not preclude the presence or addition of one or more additional features, integers, steps or components or groups thereof.

The invention is not restricted to the described embodiments in the figures, but may be varied freely within the scope of the claims.

**Claims**

1. An antenna arrangement (115) for communicating radio frequent signals with an implantable medical device, comprising:

    a patch plane (210) adapted to exchange modulated electromagnetic energy with a surrounding transmission medium, the patch plane (210) having a perimeter (a, b, c, d),
    a ground plane (220) of an electrically conducting material having an area which is larger than the patch plane (210), the ground plane (220) being located in relation to the patch plane (210) such that a perpendicular projection of the patch plane (210) onto the ground plane (220) falls entirely within the ground plane (220),
    a dielectric substrate (240) filling a volume between the patch plane (210) and the ground plane (220), the substrate (240) has a comparatively high relative permittivity and extends a well-defined distance ($L_3'$, $L_3''$) outside the volume between the patch plane (210) and the ground plane (220) with respect to at least one first segment (a, b, c) of the perimeter (a, b, c, d),

    **characterized in that**
    the arrangement comprises a grounding member (230) electrically connecting the patch plane (210) to the ground plane (220), the grounding member being attached to a second segment (d) of the perimeter (a, b, c, d), and the grounding member (230) includes a conducting plane which forms a junction (d') with the patch plane (210) along the second segment (d).

2. An antenna arrangement (115) according to claim 1, **characterized in that** the substrate (240) overlaps ($L_4'$, $H_2$) a section of the patch plane (210) along at least one sub-segment (b) of the at least one first segment (a, b, c).

3. An antenna arrangement (115) according to any one of the preceding claims, **characterized in that** the grounding member (230) is substantially perpendicular to the ground plane (220).

4. An antenna arrangement (115) according to any one of the preceding claims, **characterized in that** it comprises a signal member (310) adapted to transport a radio frequency signal between the patch plane (210) and a radio transceiver.

5. An antenna arrangement (115) according to claim 4, **characterized in that** the signal member (310) is electrically insulated from the patch plane (210).

6. An antenna arrangement (115) according to claim 4, **characterized in that** the signal member (310) is electrically connected to a side of the patch plane (210) which faces towards the ground plane (220).

7. An antenna arrangement (115) according to any one of the claims 5 or 6, **characterized in that** the signal member (310) extends through the ground plane (220) via an electrically insulated feedthrough (320) at a distance (F) from the grounding member (230).

8. An antenna arrangement (115) according to any one of the preceding claims, **characterized in that** the substrate (240) comprises a ceramic material.

9. An antenna arrangement (115) according to any one of the claims 1 - 8, **characterized in that** the substrate (240) comprises a laminated thick-film structure.

10. An antenna arrangement (115) according to claim 9, **characterized in that** the laminated thick-film structure includes at least one layer of aluminum oxide.

11. An implantable medical device, **characterized in that** it comprises an antenna arrangement (115) according to any one of the claims 1 - 10.

12. An implantable medical device according to claim 11, **characterized in that** the antenna arrangement (115) is located in a recess (710) of an outer side of a casing (111) to the device.

13. An implantable medical device according to any one of the claims 11 or 12, **characterized in that** the antenna arrangement (115) is molded into a non-conductive encapsulation (820) such that the ground plane (220) is electrically insulated from a casing (111) to the device.

14. An implantable medical device according to any one of the claims 11 or 12, **characterized in that** a casing (111) to the device constitutes the ground plane (220).

15. An implantable medical device according to any one of the claims 11 - 14, **characterized in that** the patch plane (210', 210") is tilted with respect to the ground plane (220) such that the patch plane (210', 210") is parallel to the ground plane (220) exclusively in one dimension.

16. An implantable medical device according to claim 15, **characterized in that** the antenna arrangement (115) comprises a grounding member (230) which connects the patch plane (210) electrically to the ground plane (220), and a distance between the patch plane (210', 210") and the ground plane (220)

is shortest at a point where the grounding member (230) connects to the patch plane (210', 210").

17. An implantable medical device according to claim 16, **characterized in that** the portion of the casing (111) which constitutes the ground plane (220) includes a chamfer (1010), the chamfer (1010) being oriented such that the distance between the patch plane (210") and the ground plane (220) extends gradually along a slope of the chamfer (1010) and the distance between the ground plane (220) and the patch plane (210") being largest at an edge (b) being furthest away from the grounding member (230).

18. An implantable medical device according to claim 16, **characterized in that** the substrate (240) extends over a curved side (1110) of the casing (111) such that the substrate (240) forms a tapered section towards the curved side (1110), the curved side (1110) being furthest away from the grounding member (230).

19. An implantable medical device according to any one of the claims 17 or 18, **characterized in that** the patch plane (210'") has a general curved profile with a convex face towards the ground plane (220).

**Patentansprüche**

1. Antennenanordnung (115) zum Übertragen von Radiofrequenzsignalen bei einer implantierbaren medizinischen Vorrichtung, enthaltend:

   eine Patchfläche (210), ausgelegt, modulierte elektromagnetische Energie mit einem umgebenden Übertragungsmedium auszutauschen, wobei die Patchfläche (210) einen Umfang (a, b, c, d) aufweist, eine Massefläche (220) aus einem elektrisch leitenden Material mit einer Fläche, die größer als die Patchfläche (210) ist, wobei die Massefläche (220) relativ zur Patchfläche (210) so angeordnet ist, dass eine senkrechte Projektion der Patchfläche (210) auf die Massefläche (220) vollständig in die Massefläche (220) fällt, ein dielektrisches Substrat (240), das das Volumen zwischen der Patchfläche (210) und der Massefläche (220) ausfüllt, wobei das Substrat (240) eine verhältnismäßig große relative Dielektrizitätskonstante aufweist und sich bezüglich wenigstens eines ersten Segments (a, b, c) des Umfangs (a, b, c, d) um eine eindeutig definierte Distanz $(L_3', L_3'')$ über das Volumens zwischen der Patchfläche (210) und der Massefläche (220) hinaus erstreckt, **dadurch gekennzeichnet, dass**

die Anordnung ein Erdungselement (230) enthält, das die Patchfläche (210) mit der Massefläche (220) elektrisch verbindet, wobei das Erdungselement an einem zweiten Segment (d) des Umfangs (a, b, c, d) angebracht ist und das Erdungselement (230) eine leitende Fläche enthält, die eine Verbindung (d') mit der Patchfläche (210) längs des zweiten Segmentes (d) bildet.

2. Antennenanordnung (115) nach Anspruch 1, **dadurch gekennzeichnet, dass** das Substrat (240) einen Abschnitt der Patchfläche (210) längs wenigstens eines Untersegments (b) von dem wenigstens einen ersten Segment (a, b, c) überlappt ($L_4$', $H_2$).

3. Antennenanordnung (115) nach einem der vorhergehenden Ansprüche, **dadurch gekennzeichnet, dass** das Erdungselement (230) im Wesentlichen senkrecht zur Massefläche (220) liegt.

4. Antennenanordnung (115) nach einem der vorhergehenden Ansprüche, **dadurch gekennzeichnet, dass** sie ein Signalteil (310) enthält, das ausgelegt ist ein Radiofrequenzsignal zwischen der Patchfläche (210) und einem Radiotransceiver zu befördern.

5. Antennenanordnung (115) nach Anspruch 4, **dadurch gekennzeichnet, dass** das Signalteil (310) gegenüber der Patchfläche (210) elektrisch isoliert ist.

6. Antennenanordnung (115) nach Anspruch 4, **dadurch gekennzeichnet, dass** das Signalteil (310) mit einer Seite der Patchfläche (210), die zur Massefläche (220) weist, elektrisch verbunden ist.

7. Antennenanordnung (115) nach einem der Ansprüche 5 oder 6, **dadurch gekennzeichnet, dass** sich das Signalteil (310) durch eine elektrisch isolierte Durchführung (320) in einem Abstand (F) von dem Erdungselement (230) durch die Massefläche (220) erstreckt.

8. Antennenanordnung (115) nach einem der vorhergehenden Ansprüche, **dadurch gekennzeichnet, dass** das Substrat (240) ein keramisches Material umfasst.

9. Antennenanordnung (115) nach einem der Ansprüche 1 bis 8, **dadurch gekennzeichnet, dass** das Substrat (240) einen laminierten Dickfilmaufbau umfasst.

10. Antennenanordnung (115) nach Anspruch 9, **dadurch gekennzeichnet, dass** der laminierte Dickfilmaufbau wenigstens eine Schicht aus Aluminiumoxid enthält.

11. Implantierbare medizinische Vorrichtung, **dadurch gekennzeichnet, dass** sie eine Antennenanordnung (115) nach einem der Ansprüche 1 bis 10 enthält.

12. Implantierbare medizinische Vorrichtung nach Anspruch 11, **dadurch gekennzeichnet, dass** die Antennenanordnung (115) in einer Vertiefung (710) einer Außenseite eines Gehäuses (111) zu der Vorrichtung angeordnet ist.

13. Implantierbare medizinische Vorrichtung nach einem der Ansprüche 11 oder 12, **dadurch gekennzeichnet, dass** die Antennenanordnung (115) in eine nicht leitende Einkapselung (820) so eingeformt ist, dass die Massefläche (220) gegenüber einem Gehäuse (111) zu der Vorrichtung elektrisch isoliert ist.

14. Implantierbare medizinische Vorrichtung nach einem der Ansprüche 11 oder 12, **dadurch gekennzeichnet, dass** ein Gehäuse (111) zu der Vorrichtung die Massefläche (220) darstellt.

15. Implantierbare medizinische Vorrichtung nach einem der Ansprüche 11 bis 14, **dadurch gekennzeichnet, dass** die Patchfläche (210', 210") gegenüber der Massefläche (220) so geneigt ist, dass mit Ausnahme in einer Dimension die Patchfläche (210', 210") parallel zur Massefläche (220) verläuft.

16. Implantierbare medizinische Vorrichtung nach Anspruch 15, **dadurch gekennzeichnet, dass** die Antennenanordnung (115) ein Erdungselement (230) enthält, welches die Patchfläche (210) elektrisch mit der Massefläche (220) verbindet, und der Abstand zwischen der Patchfläche (210', 210") und der Massefläche (220) an einem Punkt am kürzesten ist, an dem das Erdungselement (230) die Patchfläche (210', 210") verbindet.

17. Implantierbare medizinische Vorrichtung nach Anspruch 16, **dadurch gekennzeichnet, dass** der Abschnitt des Gehäuses (111), der die Massefläche (220) bildet, eine Abschrägung (1010) enthält, wobei die Abschrägung (1010) so ausgerichtet ist, dass die Distanz zwischen der Patchfläche (210") und der Massefläche (220) sich allmählich längs einer Neigung der Abschrägung (1010) erstreckt und der Abstand zwischen der Massefläche (220) und der Patchfläche (210") am größten an einer Kante (b) ist, die sich am weitesten vom Erdungselement (230) entfernt befindet.

18. Implantierbare medizinische Vorrichtung nach Anspruch 16, **dadurch gekennzeichnet, dass** sich das Substrat (240) über eine gekrümmte Seite (1110) des Gehäuses (111) so erstreckt, dass das Substrat (240) in Richtung zur gekrümmten Seite (1110) einen sich verjüngenden Abschnitt bildet, wobei die gekrümmte Seite (1110) sich am weitesten vom Erdungselement (230) entfernt befindet.

19. Implantierbare medizinische Vorrichtung nach einem der Ansprüche 17 oder 18, **dadurch gekennzeichnet, dass** die Patchfläche (210''') ein im Allgemeinen gekrümmtes Profil aufweist mit einer zur Massefläche (220) gerichteten konvexen Fläche.

## Revendications

1. Agencement (115) d'antenne pour échanger des signaux radioélectriques avec un dispositif médical implantable, comprenant :

   un plan (210) de plaque, conçu pour échanger une énergie électromagnétique modulée avec un milieu de transmission environnant, le plan (210) de plaque ayant un périmètre (a, b, c, d), un plan (220) de masse en un matériau conducteur de l'électricité ayant une surface qui est supérieure à celle du plan (210) de plaque, le plan (220) de masse étant positionné par rapport au plan (210) de plaque, de telle sorte qu'une projection perpendiculaire du plan (210) de plaque sur le plan (220) de masse tombe entièrement à l'intérieur du plan (220) de masse, un substrat (240) diélectrique emplissant un volume entre le plan (210) de plaque et le plan (220) de masse, le substrat (240) ayant une permittivité relative comparativement élevée et s'étendant sur une distance ($L_3'$, $L_3''$) bien définie, à l'extérieur du volume entre le plan (210) de plaque et le plan (220) de masse, par rapport au moins à un premier segment (a, b, c) du périmètre (a, b, c, d), **caractérisé en ce que** :

   l'agencement comprend un élément (230) de mise à la masse reliant électriquement le plan (210) de plaque au plan (220) de masse, l'élément de mise à la masse étant fixé à un deuxième segment (d) du périmètre (a, b, c, d), et l'élément (230) de mise à la masse comprenant un plan conducteur qui forme une jonction (d') avec le plan (210) de plaque le long du deuxième segment (d).

2. Agencement (115) d'antenne, suivant la revendication 1, **caractérisé en ce que** le substrat (240) recouvre partiellement ($L_4'$, $H_2$) une section du plan (210) de plaque, le long au moins d'un sous-segment (b) dudit au moins premier segment (a, b, c).

3. Agencement (115) d'antenne, suivant l'une quelconque des revendications précédentes, **caractérisé**

**en ce que** l'élément (230) de mise à la masse est sensiblement perpendiculaire au plan (220) de masse.

4. Agencement (115) d'antenne, suivant l'une quelconque des revendications précédentes, **caractérisé en ce qu'**il comprend un élément (310) de signalisation conçu pour transporter un signal radioélectrique entre le plan (210) de plaque et un émetteur-récepteur radioélectrique.

5. Agencement (115) d'antenne, suivant la revendication 4, **caractérisé en ce que** l'élément (310) de signalisation est isolé électriquement du plan (210) de plaque.

6. Agencement (115) d'antenne, suivant la revendication 4, **caractérisé en ce que** l'élément (310) de signalisation est connecté électriquement à un côté du plan (210) de plaque qui est orienté vers le plan (220) de masse.

7. Agencement (115) d'antenne, suivant la revendication 5 ou 6, **caractérisé en ce que** l'élément (310) de signalisation s'étend à travers le plan (220) de masse, via une traversée (320) isolée électriquement, à une distance (F) par rapport à l'élément (230) de mise à la masse.

8. Agencement (115) d'antenne, suivant l'une quelconque des revendications précédentes, **caractérisé en ce que** le substrat (240) comprend une matière céramique.

9. Agencement (115) d'antenne, suivant l'une quelconque des revendications 1 à 8, **caractérisé en ce que** le substrat (240) comprend une structure à couches épaisses stratifiées.

10. Agencement (115) d'antenne, suivant la revendication 9, **caractérisé en ce que** la structure à couches épaisses stratifiées comprend au moins une couche en oxyde d'aluminium.

11. Dispositif médical implantable, **caractérisé en ce qu'**il comprend un agencement (115) d'antenne suivant l'une quelconque des revendications 1 à 10.

12. Dispositif médical implantable, suivant la revendication 11, **caractérisé en ce que** l'agencement (115) d'antenne est positionné dans un évidement (710) d'un côté extérieur d'un boîtier (111) du dispositif.

13. Dispositif médical implantable, suivant la revendication 11 ou 12, **caractérisé en ce que** l'agencement (115) d'antenne est moulé dans une matière (820) d'enrobage non conductrice, de telle sorte que le plan (220) de masse est électriquement isolé d'un boîtier (111) du dispositif.

14. Dispositif médical implantable, suivant la revendication 11 ou 12, **caractérisé en ce qu'**un boîtier (111) du dispositif constitue le plan (220) de masse.

15. Dispositif médical implantable, suivant l'une quelconque des revendications 11 à 14, **caractérisé en ce que** le plan (210', 210") de plaque est incliné par rapport au plan (220) de masse, de telle sorte que le plan (210', 210") de plaque est parallèle au plan (220) de masse exclusivement dans une dimension.

16. Dispositif médical implantable, suivant la revendication 15, **caractérisé en ce que** l'agencement (115) d'antenne comprend un élément (230) de mise à la masse qui relie électriquement le plan (210) de plaque au plan (220) de masse, et une distance entre le plan (210', 210") de plaque et le plan (220) de masse est la plus courte en un point où l'élément (230) de mise à la masse est relié au plan (210', 210") de plaque.

17. Dispositif médical implantable, suivant la revendication 16, **caractérisé en ce que** la partie du boîtier (111) qui constitue le plan (220) de masse comprend un chanfrein (1010), le chanfrein (1010) étant orienté de telle sorte que la distance entre le plan (210") de plaque et le plan (220) de masse s'étend progressivement le long d'une pente du chanfrein (1010) et la distance entre le plan (220) de masse et le plan (210") de plaque étant la plus grande à un bord (b) qui est le plus éloigné de l'élément (230) de mise à la masse.

18. Dispositif médical implantable, suivant la revendication 16, **caractérisé en ce que** le substrat (240) s'étend sur un côté incurvé (1110) du boîtier (111), de telle sorte que le substrat (240) forme une section effilée vers le côté (1110) incurvé, le côté (1110) incurvé étant le plus éloigné de l'élément (230) de mise à la masse.

19. Dispositif médical implantable, suivant la revendication 17 ou 18, **caractérisé en ce que** le plan (210''') de plaque a un profil sensiblement incurvé dont une face convexe est orientée vers le plan (220) de masse.

**Fig. 1**

**Fig. 2**

**Fig. 3a**

**Fig. 3b**

**Fig. 3c**

**Fig. 3d**

**Fig. 3e**

**Fig. 3f**

**Fig. 3g**

**Fig. 3h**

Fig. 4

Fig. 5

Fig. 6

Fig. 7

115

820

220

111

**Fig. 8**

230

210'

240

111

220

**Fig. 9**

230

210''

240

111

1010

220

**Fig. 10**

230

210'''

240

111

1110

220

**Fig. 11**

**EP 1 362 614 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6115636 A
- US 5626630 A
- US 5861019 A

### Non-patent literature cited in the description

- **C. FURSE.** Design of an Antenna for Pacemaker Communication. *Microwaves and RF,* March 2000, 73-76